# EUROPEAN PATENT APPLICATION

(11) **EP 1 510 228 A1**
(43) Date of publication of application: **02.03.2005**
(21) Application number: 04004563.5
(22) Date of filing: 27.02.2004
(51) Int. Cl.: A61L 9/12, A61L 9/03

(54) **Scent delivery apparatus**

(30) Priority: 25.08.2003 US 648123
(71) Applicant: Hewlett-Packard Development Company, L.P., Houston, Texas 77070 (US)
(72) Inventor: Samii, Morad, La Jolla California 92037 (US)
(74) Representative: Zinkler, Franz, Dipl.-Ing.

(57) **Abstract**

A scent delivery apparatus (100) comprising a scent material carrier (112) that is movable from a scent material receiving location to a scent delivery location; a scent material transfer device (104), associated with the scent material receiving location, that transfers the scent material to the carrier; and a scent material dispersal device (106), associated with scent delivery location, that causes at least some of the scent material to be released from the carrier.

## Description

### BACKGROUND OF THE INVENTIONS

### Field of the Inventions

The present inventions are directed generally to scent delivery apparatus.

### Description of the Related Art

Conventional scent delivery apparatus house scent material and deliver the scent material into a room or other area as desired. Different scent materials (also referred to as ∼scent components") are stored and mixed, if necessary, to replicate the desired scent. The scent material, whether an individual scent component or a combination of many scent components, is typically delivered through a supply line to a device which ejects the material directly into the room or other area.

The present inventor has determined that there are a number of problems associated with conventional scent delivery apparatus. For example, the present inventor has determined that there are problems associated with residual scent material, both in the supply line, and in the area into which the scent material was delivered. The residual scent material can contaminate the scent material in future scent deliveries which, in turn, can result in a scent that is vastly different than that which was intended. Vast differences can occur even when a small amount of residual scent material is present. The present inventor has also determined that the effective life of some scent materials can be longer than desired for some applications.

### BRIEF DESCRIPTION OF THE DRAWINGS

Detailed description of embodiments of the inventions will be made with reference to the accompanying drawings.
FIG. 1 is a side, partial section view of a scent delivery apparatus in accordance with one embodiment of a present invention.
FIG. 2 is a side view of a portion of the scent delivery apparatus illustrated in FIG. 1.
FIG. 3 is a plan view of a scent material carrier strip carrying scent material in accordance with one embodiment of a present invention.
FIG. 4 is a section view showing air being forced over the scent material carrier strip illustrated in FIG. 3.
FIG. 5 is a top view of a take-up reel in accordance with one embodiment of a present invention.
FIG. 6 is a section view taken along line 6-6 in FIG. 5.
FIGS. 7A-7C are plan views of scent material carrier strips carrying scent material in accordance with exemplary embodiments of a present invention.

### DETAILED DESCRIPTION OF THE EMBODIMENTS

The following is a detailed description of the best presently known modes of carrying out the inventions. This description is not to be taken in a limiting sense, but is made merely for the purpose of illustrating the general principles of the inventions. It is noted that detailed discussions of structures that are not pertinent to the present inventions, such as ink-jet printheads and ink-jet cartridges, have been omitted forthe sake of simplicity.

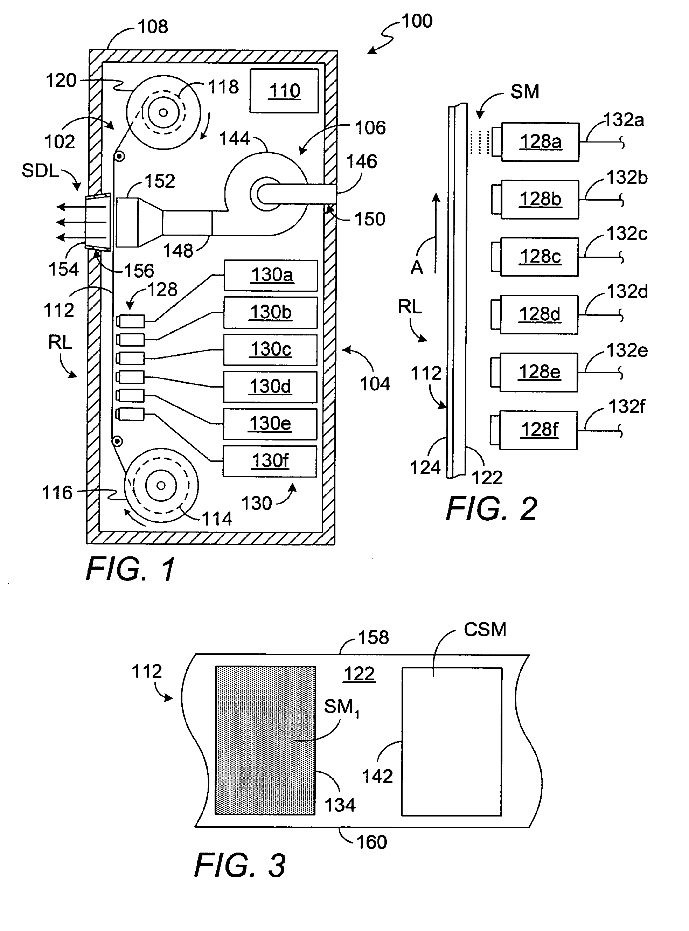

A scent delivery apparatus 100 in accordance with one embodiment of a present invention is illustrated in FIGS. 1-6. The exemplary soeent delivery apparatus 100 includes a scent material carrier system 102, a scent material transfer system 104, and a scent material dispersal system 106, all of which are located in a housing 108. A controller 110 may be used to control the operations of the scent delivery apparatus 100. As described in greater detail below, the scent material transfer system 104 deposits scent material SM onto a movable portion of the scent material carrier system 102 at a receiving location RL. The scent material SM on the movable portion of the scent material carrier system 102 is then moved to a scent delivery location SDL where the scent material dispersal system 106 causes at least some of the scent material SM to be released from the carrier system and into the room or other area that the scent delivery apparatus 100 is supplying scent material to. As a result, an olfactory experience is created for persons in the vicinity of the scent delivery apparatus 100.

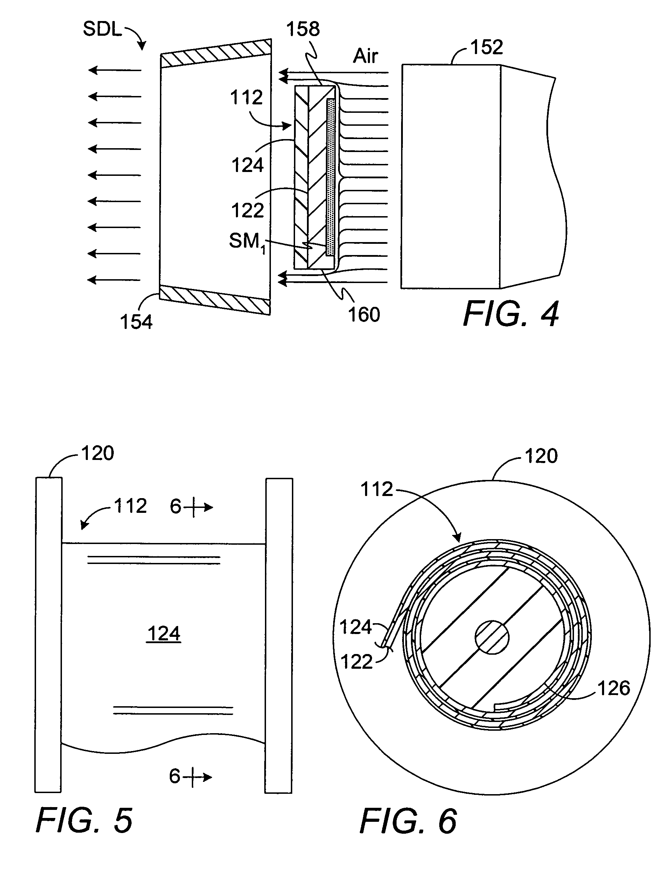

The exemplary scent material carrier system 102 includes an elongate scent carrier strip 112. The unused portion 114 of the scent carrier strip 112 is stored on a supply reel 116 that is located upstream from the receiving location RL, while the used portion 118 is stored on a take-up reel 120 that is located downstream from the scent delivery location SDL. The take-up reel 120 is preferably driven by a motor (not shown) that is controlled by the controller 110. Referring to FIGS. 2 and 4, the exemplary scent carrier strip 112 has a storage layer 122 and a vapor barrier layer 124. The scent material transfer system 104 deposits scent material SM onto the storage layer 122 at the receiving location RL. The storage layer 122 holds the scent material until some or all of the scent material forced off of the carrier strip 112 by the scent material dispersal system 106 at the scent delivery location SDL. Suitable storage layer materials include, but are not limited to, blotter and tissue paper (either coated or uncoated) and any other porous, capillary or otherwise absorbent material that will store, and then release, scent material molecules in the manner described below with reference to the soent material dispersal system 106.

The barrier layer 124 of the exemplary scent carrier strip 112 prevents any scent material SM that remains on the storage layer 122 of a particular portion of the carrier strip from escaping after that portion has passed the scent delivery location SDL and has been wound onto the take-up reel 120. More specifically, and as illustrated for example in FIGS. 5 and 6, the carrier strip 112 is oriented such that the storage layer 122 faces the inner member 126 of the take-up reel 120 and the barrier layer 124 faces outwardly. So arranged, the barrier layer 124 defines the outer surface of the carrier strip 112 as the carrier strip is wound around the take-up reel inner member 126. The barrier layer 124 is preferably formed from material that has a low moisture vapor transfer rate and is essentially impervious to the scent material SM. Suitable barrier layer materials include, but are not limited to, films formed from plastics such as polypropylenes, low density polypropylenes, polyethylenes, polyolefins, Mylar® and nylon.

The scent carrier strip 112 will typically be replaced after all of the strip has been wound onto the take-up reel 120. In one exemplary implementation, the carrier strip 112, supply reel 116 and take-up reel 120 will be housed in a small cartridge. The cartridge can be replaced, as a unit, by simply opening the housing 108, removing the used cartridge and inserting a new cartridge. Altematively, the supply reel 116 may be driven in reverse by the aforementioned motor until the carrier strip 112 is re-wound onto the supply reel. The used supply reel 116 may then be removed and replaced, and the leading end of the new carrier strip connected to the take-up reel.

Turning to the transfer of scent material SM onto the carrier strip 112 at the receiving location RL, the scent material transfer system 104 in the exemplary implementation is a spray apparatus 128 that receives scent material from a storage apparatus 130 and directs droplets of the scent material onto the carrier strip. Preferably, the spray apparatus 128 should be able to accurately meter precise quantities of scent material SM onto the carrier strip 112. The spray apparatus 128 includes a plurality of individual sprayers 128a-f, while the storage apparatus 130 includes a plurality of individual reservoirs 130a-f. The sprayers 128a-f are respectively connected to the reservoirs 130a-f by supply lines 132a-f. Each of the reservoirs 130a-f stores a different type of scent material SM and, accordingly, each of the sprayers 128a-f in the exemplary implementation sprays only one type of scent material and only one type of scent material flows through each of the supply lines 132a-f. This prevents the contamination problems that are associated with different types of scent materials flowing through the same supply line.

In the exemplary embodiment, different scent materials SM that may be used alone or in combination with one another to produce an olfactory experience are stored in the reservoirs 130a-e. The reservoir 130f stores a scent material that acts as a scent neutralizer. Suitable scent neutralizers include pine scent, lemon scent and, once developed, scent neutralizers with a neutral scent. The scent neutralizers, which typically have a short active duration, displace previous scent molecules on the olfaction scent receptors and act as an ~ofactory sensation cleanser" so that olfactory experiences produced by the exemplary scent delivery apparatus 100 are not compromised by the scent material from prior olfactory experiences that may remain in the air. The scent neutralizer material may, in some instances, be combined with other materials to form certain scents. Additionally, the scent neutralizers can be used to terminate an olfactory experience produced by the exemplary scent delivery apparatus 100 that would otherwise continue for a period that is longer than desired for the particular application.

As noted above, the scent materials SM may be used alone or in combination with one another to produce the desired olfactory experience. As such, one or more of the individual sprayers 128a-e may be used to spray the proper amount, combination and/or proportion of one or more scent materials onto particular regions of the scent carrier strip 112. These regions, which are referred to herein as ∼scent regions," are preferably (but not necessarily) rectangular, continuous and of consistent size in the exemplary embodiment illustrated in FIGS. 1-6. Referring to FIGS. 2 and 3, a first exemplary combination of scent materials SM₁, which includes scent materials SM from reservoirs 130a, 130b and 130d, is sprayed into a scent region 134. The scent material SM from reservoir 130d is sprayed onto the scent carrier strip 112 when the scent region 134 is aligned with sprayer 128d. The carrier strip 112 will then move in the direction of arrow A (FIG. 2) until the scent region 134 is aligned with sprayer 128b. Here, the scent material SM from reservoir 130b will be sprayed onto the carrier strip 112, within the scent region 134, and over the scent material from reservoir 130d. Finally, when the scent region 134 reaches the sprayer 128a, the scent material SM from the reservoir 130a will be sprayed onto carrier strip 112 and over the previously sprayed scent materials, thereby completing the first exemplary combination of scent of materials SM₁.

In some implementations of the exemplary scent delivery apparatus 100, the carrier strip 112 will be provided with encoding (such as printed indicia or holes) for system control purposes. Here, the scent delivery apparatus 100 will also include a reader or counter so that the position of the carrier strip 112, as well as the position of the individual scent regions formed thereon, can be monitored for use in the control of the scent material carrier system 102, scent material transfer system 104, and scent material dispersal system 106. The carrier strip monitoring capability may also be used to determine when the supply reel 116 is almost empty so that the user may be notified with, for example, an audible or visible indicator.

The overall amount of scent material SM in the scent region 134, as well as the relative amounts of the scent materials that are combined with one another, may be varied by, for example, varying the drop density of the spray of each material. Spraying fewer drops per unit area results in less scent material within the unit area, as compared to another spray with the same size drops and more drops per unit area. Varying the amount of scent material SM is also one way to vary the intensity of the associated olfactory experience. It should also be noted that the density of scent material may be varied along the length of a scent region, especially those which are relatively long, in order to provide an olfactory experience that varies in intensity overtime.

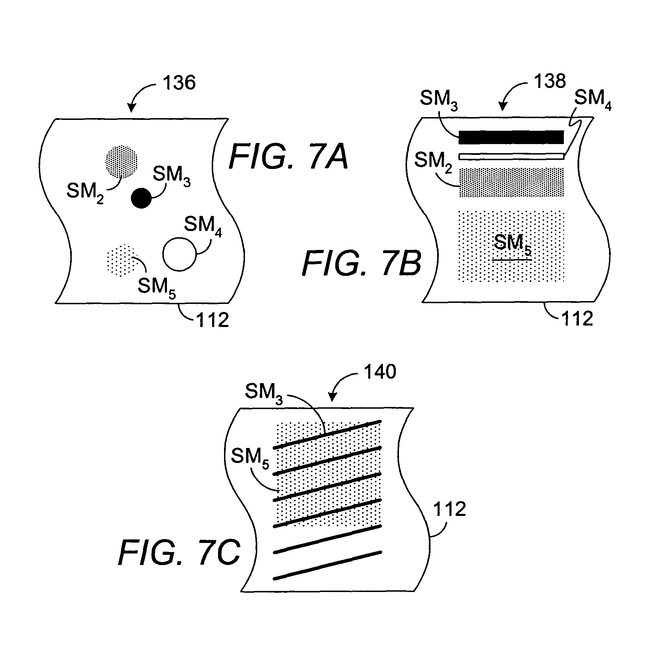

The spray apparatus 128 may also be used to form scent regions with configurations other than that illustrated in FIG. 3. A number of alternative configurations are illustrated in FIGS. 7A-C. By way of example, but not limitation, the spray apparatus 128 may be used to form the scent region 136 illustrated in FIG. 7A, which consists of a plurality of spaced deposits of scent materials SM₂-SM₅. Each of these deposits may be in the form of a single scent component, or a number of scent components in a predetermined combination. The relative amounts of the individual scent materials may be varied by varying the size of each deposit. There may be some overlap if necessary or desired. Additionally, although the exemplary deposits are circular in shape, any suitable shape may be employed. The exemplary scent region 138 illustrated in FIG. 7B also includes a plurality of spaced deposits of scent materials SM₂-SM₅. Here, however, the deposits are in the form of generally linear deposits that extend across the length of the exemplary scent region 138. The relative amounts of the scent materials SM₂-SM₅ may be varied by varying the width of the linear deposits, or by shortening the length of the deposits. There may also be instances where it is desirable to mix some, but not all, of two or more scent materials (or scent material combinations) that are used to form a scent region. Referring to FIG. 7C, the exemplary scent region 140 includes an underlying layer of scent material SM₅ and a number of linear deposits of scent material SM₃. A small portion of the scent material SM₅ is covered by the linear deposits of scent material SM₃, while a portion of the scent material SM₃ is deposited directly onto the carrier strip 112 and away from the scent material SM₅. As such, the scent region 140 will include unmixed scent material SM₃, unmixed scent material SM₅, and a mixture of the two.

As noted above, reservoir 132f may be used to store olfactory sensation cleansing material. In the exemplary embodiment illustrated in FIGS. 1-6, an olfactory sensation cleansing region 142 (FIG. 3) may be formed on the carrier strip 112, preferably between each scent region, by spraying cleansing scent material CSM onto the carrier strip with sprayer 128f.

With respect to the sprayers 128a-f themselves, and although the sprayers may be any suitable device for spraying droplets of scent material onto carrier strip 112, the sprayers in the exemplary implementation are in the form of ink-jet printheads, such as thermal, piezo, continuous or electromagnetic ink-jet printheads. The sprayers preferably, as noted above, should be capable of accurately metering the scent material. Other types of sprayes, such as air brushes and venturi meters and sprayers, may also be employed, as may any other device which can deposit scent material onto the carrier strip in manner other than spraying. Such devices include, but are not limited to, offset and lithographic printers and other common printing apparatus. Additionally, although the exemplary implementation includes six (6) sprayers and six (6) reservoirs, these numbers may be increased or decreased as necessary in order to suit the intended application. The sprayers 128a-f may also be fixed in place, or movable relative to the carrier strip 112, e.g. in a direction transverse to arrow A, and the effective size of the sprayers may be varied to suit the intended application. Additionally, in those instances where the sprayers 128a-f are in the form of ink-jet printheads, the frequency and firing pattern of the nozzles may be varied to suit the intended application.

The exemplary reservoirs 130a-f may be refillable receptades, which have an inlet for refilling, or replaceable cartridges, which may be removed from the housing 108 and replaced. In those instances where the scent delivery apparatus 100 is intended to be used for a limited time and then discarded, the reservoirs 130a-f could be configured such that they are not refillable or replaceable. The reservoirs 130a-f may also be combined with sprayers 128a-f into individual integral units that may be replaced as needed in a manner similar to the cartridges in an ink-jet printer. A suitable ink-jet cartridge that may be used to deposit scent material is the No. 45 thermal ink-jet cartridge from the Hewlett-Packard Company.

Turning to the scent material dispersal system 106, and referring to FIGS. 1 and 4, the exemplary scent delivery apparatus 100 is provided with an air pump 144 that has an inlet 146 and an outlet 148. The air pump inlet 146, which may include an air filter, is associated with an inlet opening 150 in the housing 108. The air pump outlet 148 is connected to a vent 152 which faces the scent delivery location SDL. The vent 152 directs air from the air pump 144 through the scent delivery location SDL, over and around the carrier strip 112, and through a guide 154 that is located in an opening 156 in the housing 108. The carrier strip 112 is oriented such that the scent material SM (or cleansing scent material CSM) faces the vent 152.

When a scent region, such as scent region 134, reaches the scent delivery location SDL, the air being forced through the vent 152 by the air pump 144 will force some (and preferably all) of the scent material molecules off of the carrier strip 112. Referring to FIGS. 1 and 4, the air and scent material molecules will be forced around the lateral edges 158 and 160 of the carrier strip, through the guide 154 and into the room or other area that is to receive the olfactory experience. To that end, the vent 152 and guide 154 are preferably sized and configured such that the length of the scent delivery location SDL (measured in the direction of arrow A in FIG. 2) will be larger than the length of the scent region, and the width of the scent delivery location will be larger that the width of the carrier strip 112. In addition to insuring that all of the scent material SM that is driven off of the carrier strip 112 escapes from the housing 108, this configuration will also prevent the scent material associated with one scent region from contaminating subsequent olfactory experiences by insuring that the scent material will not come to rest on the guide 154.

The present inventions are not limited to scent material dispersal systems with an air pump. Other apparatus, such as fans, blowers, impellers and turbines, may be used to drive air and scent material through the scent delivery location SDL. The air pump 144 (or other apparatus) may be run intermittently or continuously depending on the application. The air speed and volume through the guide 154 may also be varied, either by controlling the air pump or with dampers, in order to more precisely control the olfactory experience.

The exemplary scent delivery apparatus 100 may be used to provide a metered (in type, intensity and duration) delivery of scent material for olfactory experiences in a wide variety of applications.

The scent delivery apparatus may be used, for example, in supermarket kiosks that allow customers to smell particular products either on demand or automatically when the customer approaches a particular item. When a customer approaches a particular type of food, such as apple pies, the scent delivery apparatus 100 may be used to supply an ∼apple pie "olfactory experience in order to trigger a memory in the consumer's mind and motivate the consumer to buy the apple pie. More specifically, when a sensor in the kiosk determines that a consumer is approaching (or when a consumer activates the kiosk), the scent material transfer system 104 will transfer a scent material (or combination of scent materials) that replicates the scent of an apple pie onto the carrier strip 112 at the receiving location RL. This forms an ∼apple pie"scent region. The ∼apple pie"scent region will then be moved to the scent delivery location SDL, where the scent material dispersal system 106 will force the scent material off of the carrier strip 112 and out of the kiosk. The olfactory experience produced by the scent delivery apparatus 100 can end in a variety of ways. For example, the scent material may be allowed to replicate the ∼apple pie"smell for as long as its effective life allows. Alternatively, the apparatus 100 may be used to deliver cleansing scent material after a predetermined interval or when the sensor determines that the consumer has walked away from the kiosk. The cleansing scent material will be transferred to the carrier strip 112 in the receiving location RL and then forced off of the carrier strip and out of the kiosk at the scent delivery location SDL in the manner described above.

Another exemplary application of the present scent delivery apparatus 100 is theaters and cinemas. An olfactory experience track that follows the play or movie may be used to produce particular olfactory experiences at predetermined times during the movie, and end those experiences at predetermined times so that future olfactory experiences are not compromised. Here too, the scent material (or combination of scent materials) would be transferred to the carrier strip 112 in the receiving location RL and then forced off of the carrier strip and out of the kiosk at the scent delivery location SDL. The theater would preferably be provided with a plurality of scent delivery apparatuses 100.

Mood enhancement systems are another exemplary application of the present inventions. Such systems may be used to increase awareness (in an airplane cockpit, for example), assist relaxation, or to produce psychosomatic effects on the central nervous system. The present inventions may also be employed in bathrooms, hospitals and stores of types other than the aforementioned supermarket.

Although the present inventions have been described in terms of the embodiments above, numerous modifications and/or additions to the above-described embodiments would be readily apparent to one skilled in the art. It is intended that the scope of the present inventions extend to all such modifications and/or additions.

## Claims

1. An apparatus for delivering scent material, comprising:
a carrier (112) that is movable from a scent material receiving location to a scent delivery location;
a scent material transfer device (104), associated with the scent material receiving location, that transfers the scent material to the carrier; and
a scent material dispersal device (106), associated with scent delivery location, that causes at least some of the scent material to be released from the carrier.

2. An apparatus as claimed in claim 1, wherein the carrier comprises (112) an absorbent material.

3. An apparatus as claimed in claim 1, wherein the carrier (112) comprises paper.

4. An apparatus as claimed in claim 1, wherein the carrier (112) comprises an elongate strip of carrier material, the apparatus further comprising:
a supply reel (116), located upstream from the scent material transfer device (104), that stores unused portions of the elongate strip of carrier material; and
a take-up reel (120), located downstream from the scent material dispersal device (106), that receives used portions of the elongate strip of carrier material after the dispersal device has caused at least some the scent material to be released from the elongate strip of carrier material.

5. An apparatus as claimed in claim 4, wherein the carrier (112) comprises a storage layer (122) and a barrier layer (124) and the carrier is arranged such that the storage layer faces the take-up reel (120) and the barrier layer faces away from the take-up reel.

6. An apparatus as claimed in claim 1, wherein the scent material transfer device (104) comprises a plurality of scent material transfer devices (128) associated with respective portions of the scent receiving location.

7. An apparatus as claimed in claim 6, wherein the scent material comprises a plurality of different scent materials, the apparatus further comprising:
a plurality of scent material reservoirs (130) respectively connected to the plurality of scent material transfer devices (128).

8. An apparatus as claimed in claim 1, wherein the scent material transfer device sprays droplets of scent material onto the carrier.

9. An apparatus as claimed in claim 1, wherein the scent material dispersal device (106) drives air through the scent delivery location.

10. An apparatus as claimed in claim 1, further comprising:
a housing (108), in which the carrier (112), the scent material transfer device (104), and the scent material dispersal device (106) are located, including an outlet (156);
wherein the scent material dispersal device drives air through the scent delivery location and through the outlet.
